# EUROPEAN PATENT APPLICATION

(11) **EP 4 438 619 A1**
(43) Date of publication of application: **02.10.2024**
(21) Application number: 21967933.9
(22) Date of filing: 27.12.2021
(51) Int. Cl.: C07K 14/47, C12N 15/12, C07K 19/00, C12N 15/62, A61K 38/17, A61K 47/64, A61K 47/68, A61P 35/00

(54) **FUSION PROTEIN, AND PREPARATION METHOD THEREFOR AND USE THEREOF**

(30) Priority: 13.12.2021 CN 202111518468
(71) Applicant: Sun Yat-Sen University Cancer Center (SYSUCC), Guangzhou Guangdong 510060 (CN)
(72) Inventor: ZENG, Musheng, Guangzhou, Guangdong 510060 (CN); FENG, Guokai, Guangzhou, Guangdong 510060 (CN); LI, Ziqian, Guangzhou, Guangdong 510060 (CN); YANG, Jie, Guangzhou, Guangdong 510060 (CN); YE, Jiacong, Guangzhou, Guangdong 510060 (CN)
(74) Representative: Gassner, Birgitta
(86) International application number: PCT/CN2021/141426
(87) International publication number: WO 2023/108803

(57) **Abstract**

Provided are a fusion protein, and a preparation method therefor and the use thereof. The fusion protein comprises a GSDME mutant protein and a tumor cell-specific cell-penetrating peptide. In the GSDME mutant protein, a tumor-targeting effect is provided by mutating a sequence recognized by cysteine-aspartic proteases in GSDME protein into an amino acid sequence recognized by matrix metalloproteinase; and the in-vitro misfolding rate of GSDME protein is reduced by means of mutating cysteine into alanine, increasing the druggability of the protein. The fusion protein which has a tumor targeting property, penetrating property and tumor microenvironment-specific activation is constructed, which provides a new idea for the development of targeted anti-cancer drugs; and the N terminus of pyroptosis component GSDME can be directly delivered into tumor cells to induce pyroptosis and the sensitivity of other anti-tumor immunotherapies is improved by means of activating the body's autoimmunity, improving the efficiency and expanding the applicability of existing anti-tumor immunotherapies, which is of great significance.

## Description

### TECHNICAL FIELD

The present invention belongs to the field of biomedicines, and particularly, to a fusion protein, and a preparation method therefor and use thereof.

### BACKGROUND

The gasdermin-E (GSDME) gene, also known as DFNA5, encodes 496 amino acids, contains a gasdermin domain, and is one of gasdermin family members. The GSDME protein consists of two domains with different functions. The N-terminal has the membrane perforation activity, and the C-terminal maintains its conformational stability and transformation through domain-domain interaction with the N-terminal, thereby exerting biological functions of self-inhibition and regulating pyroptosis.

The pyroptosis is a "burning" programmed cell death accompanied by inflammatory response. The inflammasome or lipopolysaccharide (LPS) and the like activate inflammatory caspase including cysteine or granzyme to cut gasdermin, thus forming N-terminal and C-terminal domains. The N-terminal is self-assembled into a barrel structure and directly binds to membrane lipids to induce membrane perforation, which destroys the intracellular osmotic pressure, causes cells to swell, releases lactate dehydrogenase (LDH), and causes the inflammatory response. At the same time, with the release of cytokines interleukin IL-1 and IL-18, a further inflammatory response will be caused. Under physiological conditions, GSDME can be cut and activated by Caspase-3/Granzyme B, leading to conversion from apoptosis to pyroptosis. As early as decades ago, studies showed that the promoter of GSDME was methylated in a variety of tumor cells, and this epigenetic modification inhibited its expression in tumor cells. GSDME can inhibit the formation and proliferation of tumor cell colonies such as gastric cancer, melanoma, colorectal cancer, etc.. And the reduction of the GSDME level is associated with the increase of the breast cancer mortality. These clues suggest that GSDME is a tumor suppressor. In recent years, with the discovery of the role of GSDME in pyroptosis, more and more studies have confirmed that GSDME is an effective tumor suppressor gene. However, it is silently expressed or mutated in most tumor tissues, resulting in loss of function. When GSDME in tumors is activated, it can transform "cold" tumors that an immune system cannot recognize into "hot" tumors that the immune system can control.

Tumors have many ways to evade attack by the immune system, and scientists have also fought back through cancer immunotherapy. Currently, the main method is to use immune checkpoint inhibitors, and another method is chimeric antigen receptor T cell-immunotherapy (CAR-T). However, not all cancer patients can be treated with these methods, studies have shown that these methods are only effective for a few cancer types, and most tumors are "cold" tumors, resulting in certain limitations of these immunotherapies. GSDME is a molecular switch that initiates pyroptosis and innate immunoregulation. A fusion protein method is chosen to initiate pyroptosis because, compared with small molecule drugs, protein drugs have high activity, strong specificity, low toxicity, clear biological functions and convenient production due to prokaryotic or eukaryotic expression, which is conducive to large-scale production and clinical application. Based on the mechanism of action of GSDME, the present invention uses a fusion expression to construct a protein drug system that targets tumors and activates pyroptosis, and a series of functional experiments are used to verify the effectiveness of tartrate resistant acid phosphatase (TRAP) in inducing pyroptosis.

### SUMMARY

The purpose of first aspect of the present invention is to provide a GSDME mutant protein.

The purpose of second aspect of the present invention is to provide a related biological material of the GSDME mutant protein in the first aspect of the present invention.

The purpose of third aspect of the present invention is to provide a fusion protein.

The purpose of fourth aspect of the present invention is to provide a related biological material of the fusion protein in the third aspect of the present invention.

The purpose of fifth aspect of the present invention is to provide a preparation method of the fusion protein in the third aspect of the present invention.

The purpose of sixth aspect of the present invention is to provide use of the GSDME mutant protein in the first aspect of the present invention or the related biological material in the second aspect of the present invention or the fusion protein in the third aspect of the present invention or the related biological material in the fourth aspect of the present invention.

The purpose of seventh aspect of the present invention is to provide a product.

The purpose of eighth aspect of the present invention is to provide a combined medicine.

The technical solutions adopted by the present invention are:
In the first aspect of the present invention, a GSDME mutant protein is provided, wherein the GSDME mutant protein based on GSDME is at least one of:
a) a cysteine in SEQ ID NO: 1 is mutated to alanine; and/or
b) a cysteine in SEQ ID NO:1 is mutated to other amino acids and having the same or similar function as a); and/or
c) a DMPDAAH amino acid sequence recognized by caspase3 in SEQ ID NO: 1 is mutated to an amino acid sequence recognized by matrix metalloproteinases (MMPs).

In some embodiments, one or more mutations at cysteine positions in SEQ ID NO: 1 are specifically at C45, C159, C168, C180, C235, C331, C365, C371, C407, C408, C417, C482, C489; and preferably all cysteines are mutated to alanine.

In some embodiments of the present invention, the MMPs are at least one of MMPs selected from the group consisting of MMP1, MMP2, MMP9 and MMP11.

In some embodiments of the present invention, the sequence of the GSDME mutant protein is as shown in SEQ ID NO:2 or SEQ ID NO:6, preferably SEQ ID NO:6.

The second aspect of the present invention, the related biological material of the GSDME mutant protein in the first aspect of the present invention is provided, wherein the related biological material is any one of the following (A1) to (A8):
(A1) a nucleic acid molecule encoding the GSDME mutant protein;
(A2) an expression cassette containing the nucleic acid molecule in (A1);
(A3) a recombinant vector containing the nucleic acid molecule in (A1);
(A4) a recombinant vector containing the expression cassette in (A2);
(A5) a recombinant microorganism containing the nucleic acid molecule in (A1);
(A6) a recombinant microorganism containing the expression cassette in (A2);
(A7) a recombinant microorganism containing the recombinant vector in (A3); and
(A8) a recombinant microorganism containing the recombinant vector in (A4).

The third aspect of the present invention, the fusion protein containing GSDME protein and a tumor cell-specific membrane-penetrating peptide is provided.

In some embodiments of the present invention, the GSDME protein is the GSDME mutant protein in the first aspect of the present invention or protein shown in SEQ ID NO: 1.

In some embodiments of the present invention, the tumor cell-specific membrane-penetrating peptide is a protein polypeptide, a single-chain variable region antibody or a nanobody.

In some embodiments of the present invention, the protein polypeptide is H16; the single-chain variable region antibody is Her2 single-chain variable region 4D5; and the nanobody is Her2 nanobody.

In some embodiments of the present invention, the tumor includes at least one of pancreatic cancer, colorectal cancer, nasopharyngeal cancer, renal cancer, lung cancer, liver cancer, breast cancer, prostate cancer, gastrointestinal cancer, peritoneal cancer, melanoma, endometrial cancer, ovarian cancer, cervical cancer, uterine cancer, bladder cancer, glioblastoma, brain metastases, salivary gland cancer, thyroid cancer, brain cancer, lymphoma, myeloma and head and neck cancer.

In some embodiments of the present invention, the amino acid sequence of the fusion protein is a) or b):
a) as shown in any one of SEQ ID NO:3-4 or SEQ ID NO:7-8; and
b) the amino acid sequence shown in a) configured to be modified by one or more amino acid substitutions, deletions or additions and has the same or similar function.

In some embodiments of the present invention, the amino acid sequence of the fusion protein is as shown in SEQ ID NO: 9 or SEQ ID NO: 10.

The fourth aspect of the present invention provides the related biological material of the fusion protein according to the third aspect of the present invention, where the related biological material is any one of the following (B 1) to (B8):
(B 1) a nucleic acid molecule encoding the fusion protein;
(B2) an expression cassette containing the nucleic acid molecule in (B 1);
(B3) a recombinant vector containing the nucleic acid molecule in (B 1);
(B4) a recombinant vector containing the expression cassette in (B2);
(B5) a recombinant microorganism containing the nucleic acid molecule in (B1);
(B6) a recombinant microorganism containing the expression cassette in (B2);
(B7) a recombinant microorganism containing the recombinant vector in (B3); and
(B8) a recombinant microorganism containing the recombinant vector in (B4).

In some embodiments of the present invention, the microorganism may be a prokaryotic microorganism or a eukaryotic microorganism.

The fifth aspect of the present invention, the preparation method for the fusion protein according to the third aspect of the present invention is provided, and the fusion protein is obtained by cultivating the recombinant microorganism in the fourth aspect of the present invention.

The sixth aspect of the present invention, the use of the mutant protein in the first aspect of the present invention or the related biological material of the GSDME mutant protein in the second aspect of the present invention or the fusion protein in the third aspect of the present invention or the related biological material of the fusion protein in the fourth aspect of the present invention in the preparation of a product is provided.

In some embodiments of the present invention, the function of the product is any one of (1) to (5):
(1) tumor targeting;
(2) inducing pyroptosis;
(3) tumor cell-specific membrane-penetrating;
(4) activating the body autoimmunity; and
(5) anti-tumor.

In some embodiments of the present invention, the product is a medicine.

The seventh aspect of the present invention, the product comprising the mutant protein in the first aspect of the present invention or the related biological material of the GSDME mutant protein in the second aspect of the present invention or the fusion protein in the third aspect of the present invention or the related biological material of the fusion protein in the fourth aspect of the present invention is provided.

In some embodiments of the present invention, the product is a medicine.

In some embodiments of the present invention, the medicine may also contain pharmaceutically acceptable excipients.

The eighth aspect of the present invention, a combined medicine is provided, comprising at least one of the medicines in the seventh aspect of the present invention, and a tumor-targeting drug.

The beneficial effects of the present invention are:
The present invention first provides a GSDME mutant protein, a DMPDAAH amino acid sequence recognized by caspase3 in the GSDME protein is mutated to the amino acid sequence recognized by MMPs to provide a targeting effect on tumors, mutation of cysteine to alanine reduces misfolding rate of the GSDME protein in vitro and increases druggability possibility thereof; and based on the GSDME mutant protein, the fusion protein is constructed, the tumor cell-specific membrane-penetrating peptide is also contained, and the fusion protein with the tumor targeting property, penetrability and tumor microenvironment-specific activation is constructed. The fusion protein with the tumor targeting property, penetrability and tumor microenvironment-specific activation is designed and constructed first for activating the anti-tumor immune protein GSDME in the present invention, which provides a new idea for the development of targeted anti-cancer drugs. This fusion protein can directly deliver the N-terminal of the pyroptosis component GSDME into tumor cells to induce pyroptosis, and at the same time, this protein can improve the sensitivity of other anti-tumor immunotherapies by activating the body autoimmunity, which expands the efficiency and scope of application of existing anti-tumor immunotherapies and is of great significance in tumor treatment.

Compared with the technology of finding and using small molecules to activate GSDME, the fusion protein TRAP provided by the present invention is simple in synthesis, convenient to produce due to prokaryotic or eukaryotic expressions, high in acting efficiency, and is easier to be produced on a large scale and commercially applied.

### BRIEF DESCRIPTION OF DRAWINGS

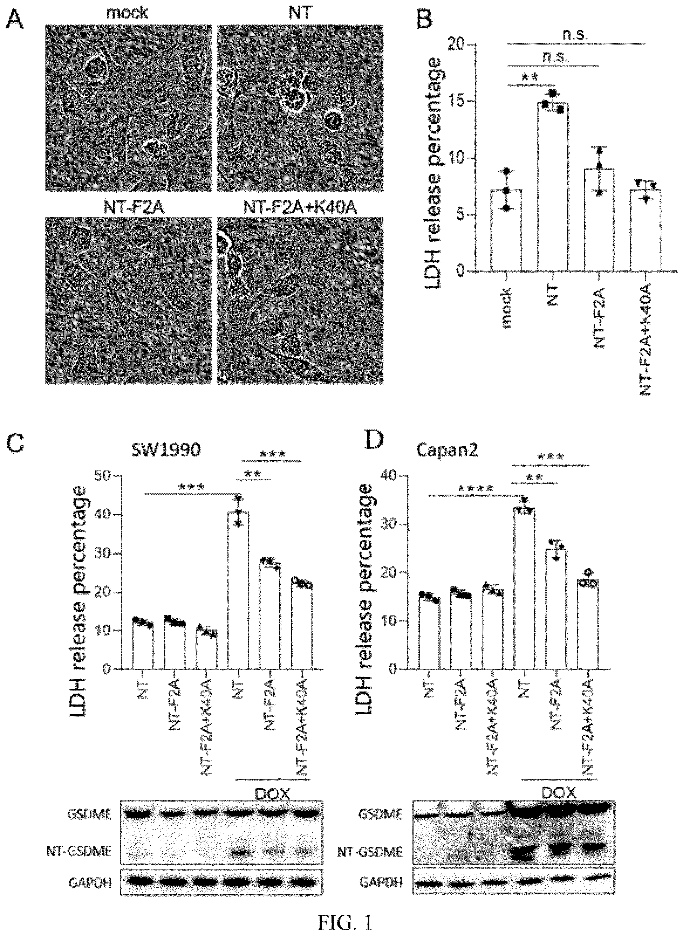

FIG. 1 is a result diagram of pyroptosis induced at N-terminal of GSDME protein, where FIG. 1A is a result diagram of cellular morphology after SW1990 cells are transiently transfected by NT, NT-F2A and NT-F2A+K40A plasmids; FIG. 1B is a result diagram of cell LDH release percentage after SW1990 cells are transiently transfected by NT, NT-F2A and NT-F2A+K40A plasmids; FIG. 1C is LDH release percentage result diagram and NT expression situation diagram after DOX induces SW1990 cells to express NT, NT-F2A and NT-F2A+K40A; and FIG. 1D is LDH release percentage result diagram and NT expression situation diagram after DOX induces Capan 2 cells to express NT, NT-F2A and NT-F2A+K40A.
FIG. 2 is an expression result diagram of MMP11 in tumor tissues and cells, where FIG. 2A is statistical result diagram of the expression of MMP11 at different tumors and normal tissues in GEPIA2 database; and FIG. 2B is the expression result diagram of MMP11 at a cellular level.

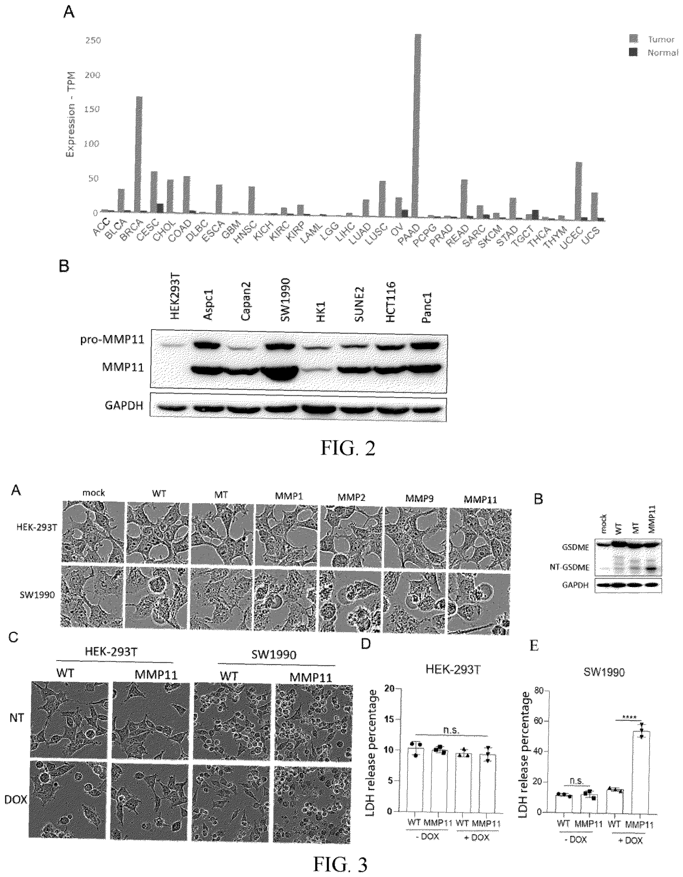

FIG. 3 is a result diagram of inducing pyroptosis after caspase-3 restriction enzyme cutting site of GSDME protein is mutated to MMPs restriction enzyme cutting site, where FIG. 3A is a result diagram of cellular morphology after HEK-293T and SW1990 cells are transiently transfected by GSDME WT, MT and MMPs plasmids; FIG. 3B is an expression result diagram of full-length and N-terminal spliceosome of cell GSDME after SW1990 cells are transiently transfected by GSDME WT, MT and MMP11 plasmids; FIG. 3C is a result diagram of cellular morphology after DOX induces HEK-293T and SW1990 cells to express GSDME WT or MMP11; FIG. 3D is a LDH release percentage result diagram after OX induces HEK-293T cells to express GSDME WT or MMP11; and FIG. 3E is a LDH release percentage analysis result diagram after OX induces SW1990 cells to express GSDME WT or MMP11.
FIG. 4 is a construction schematic diagram of TRAP system and a process schematic diagram of inducing pyroptosis.
FIG. 5 is a purification effect diagram of H16-MMP11 protein.
FIG. 6 is an effect diagram of H16-MMP11 protein inducing pyroptosis in HEK-293T, SUNE2, SW1990 and HCT116.
FIG. 7 is a purification effect diagram of 4D5-MMP11 protein.
FIG. 8 is an effect diagram of 4D5-MMP11 protein inducing pyroptosis in MCF7, SKBR2 and SW1990, where FIG. 8A is a cellular morphology diagram after MCF7, SKBR2 and SW1990 cells are treated with 4D5-MMP11 protein; and FIG. 8B is a LDH release percentage result diagram after MCF7, SKBR2 and SW1990 cells are treated by 4D5-MMP11 protein.
FIG. 9 is a protein activity detection result diagram after GSDME amino acid mutation modification, where FIG. 9A is cellular morphology result diagram after transient transduction of GSDME NT, NT F2A+K40A, NT C-A and NT C-S; FIG. 9B is an expression result diagram of full-length and N-terminal spliceosome of cell GSDME after SW1990 is transiently transfected by GSDME NT, NT F2A+K40A, NT C-A and NT C-S; and FIG. 9C is a cell LDH release percentage result diagram after SW1990 is transiently transfected by GSDME NT, NT F2A+K40A, NT C-A and NT C-S.
FIG. 10 is a purification effect diagram of sumo-H16-WT C-A and sumo-H16-MMP11 C-A proteins.
FIG. 11 is an effect diagram of sumo-H16-WT C-A and sumo-H16-MMP11 C-A proteins inducing pyroptosis in SW1990 and Capan2, where FIG. 11A is cellular morphology diagram after sumo-H16-WT C-A and sumo-H16-MMP11 C-A proteins are treated with SW1990 and Capan2 cells, respectively; and FIG. 11B is a LDH release percentage result diagram after sumo-H16-WT C-A and sumo-H16-MMP11 C-A proteins are treated with SW1990 and Capan2 cells, respectively.

Among them: **** means P<0.0001; ** indicates P<0.01; *** indicates P<0.001; and ns means no significant difference.

### DETAILED DESCRIPTION

The concepts and the generate technical effects of the present invention are clearly and completely described below in combination with examples, so as to fully understand the purposes, features and effects of the present invention. It is apparent that the described examples are only a part of the examples of the present invention but not all. Based on the examples of the present invention, all the other examples obtained by those of ordinary skill in the art on the premise of not contributing creative effort should belong to the protection scope of the present invention.

### Example 1 Construction of TRAP system and effect verification thereof

In this example, the designed and constructed tumor responsive activable pyroptosis (TRAP) system to target tumor cells and induce pyroptosis was taken as an example, to demonstrate and confirm the feasibility and advantages of the technical solution of the present invention. The protein GSMDE with a pore-forming effect is a tumor suppressor gene and participates in anti-tumor immunity. Therefore, when designing drugs, the N-terminal of the GSDME protein effect is brought into cells to induce pyroptosis, which is of great significance for the clinical treatment of tumors. However, there are currently no drugs targeting GSDME for clinical treatment.

### I. Preparation of fusion protein TRAP molecules

### (I) Design and construction of TRAP molecular tools

### 1. Verification of the action of the N-terminal of GSDME protein effect

The action of the N-terminal of GSDME protein in self-assembly in cells and inducing pyroptosis was verified through this experiment. The transient transfection and Retro-X Tet-On 3G induced expression system were mainly used to detect the effect of the N-terminal of the GSDME protein in inducing pyroptosis, with the specific steps as follows:
1.1. A DNA sequence containing the molecular components of GSDME N-terminal, N-terminal F2A mutation and N-terminal F2A+K40A mutation was synthesized and cloned into a mammalian expression vector pcDNA3.1/pRetro-X 3G (plasmid was synthesized and constructed by Suzhou Hongxun Biotechnology Co., Ltd.). The synthesized plasmid was transformed into DH5α competent cells (100 ng plasmid was mixed with 50 µL of competent cells, heat shocked for 90 s at 42°C), and the positive recombinants were screened out through an ampicillin-resistant plate and subjected to colony PCR verification and DNA sequencing verification, and sequencing results were analyzed through NCBI BLAST comparison. Plasmids were stored for long-term at -20°C, and correctly sequenced strains were added to 15% glycerol and stored at -80°C.
1.2. Transiently transfected GSDME NT (N-terminal fragment of GSDMD) to verify pyroptosis-inducing effect thereof
1.2.1. Human pancreatic cancer cells SW1990 were spread in a 96-well plate, the cells were divided into 4 groups, with four duplicated wells in each group, 100 µL of cell suspension was added to each well at a concentration of 2×10⁴ cells/mL; and at the same time, 100 µL of phosphate buffer saline (PBS) was added around the 96-well cell culture plate to prevent the cell culture medium from evaporating, and cultivation was performed for 24 h at 37°C in a 5% CO₂ incubator to adhere to the wall.
1.2.2. The next day, pcDNA3.1-GSDME NT/NT-F2A/NT-F2A+K40A plasmids were transfected into the cells (the usage ratio was 2 µL PEI + 1 µg plasmid) using polyethylenimine (PEI), long-time cell living imaging IncuCyte was used to observe the change of the cellular morphology, after 6 h, the shooting was completed and the cells were collected to detect the expression of the GSDME NT protein by western blot (WB), and the cell supernatant was collected to detect the release of lactate dehydrogenase (LDH).
1.2.3. 80 µl of the cell supernatant from the 96-well plate was transferred into a new 96-well plate, and a lactate dehydrogenase cytotoxicity detection kit (Cat. No.: C0017; purchased from: Beyotime) was used to detect the LDH release percentage.
1.2.4. The cells in the 96-well plate were digested and collected into 1.5 mL EP tubes, and 20 µL of cell lysis solution was added to each tube (Cat. No.: P0013G; purchased from: Beyotime). Lysis was performed on ice, vortex oscillation was performed once every 5 min, after lysis for 30 min, the cells are centrifuged for 30 min at 12000 rpm and at 4°C, and the supernatant was collected in a new 1.5 mL EP tube.
1.2.5. The bicinchoninic acid (BCA) method was used to measure the protein concentration, and 30 µg of protein was taken from each group for sodium dodecyl sulfate (SDS) denaturing gel electrophoresis, after transferred and blocked with 5% skim milk for 1 h, NT-GSDME (source: Abcam; Cat. No.: ab215191), GAPDH (source: Bioworld; Cat. No.: AP0063) primary antibody were added according to the dilution at a ratio of 1: 2000, the mixture was shaken at 4°C overnight to remove antibodies, then the membrane was washed for 3 times with TBST, with 10 min for each time; the mixture was diluted with corresponding HRP-labeled rabbit secondary antibodies (source: TransGen; Cat. No.: HS101-01) at a ratio of 1:3000 and shaken and incubated for 1 h at room temperature, and the membrane was washed for 3 times with TBST, with 10 min for each time; an ELC luminescence kit was developed in a dark room; and all primary antibody concentrations were 1:2000 (1 µg protein was diluted in 2000 µL primary antibody diluent).
1.2.6. The results are as shown in FIGs. 1A and 1B, and after transient transfection with GSDME NT, cells underwent pyroptosis.
1.3. Retro-X Tet-On induced expression system verified the effect of GSDME NT on inducing pyroptosis
1.3.1. According to the operating steps of the Retro-X Tet-On induced expression system, GP2-293 cells were used to package and collect the vector pRetro-X 3G lentivirus containing the target gene and the lentivirus containing the Tet-On promoter component, respectively.
1.3.2. The human pancreatic cancer cell Capan2 and SW1990 cells were spread in a six-well plate, with 500,000 cells in each well. After cell adherence, the lentivirus containing the target gene and the lentivirus containing the Tet-On promoter component were added into the six-well plate at a ratio of 1:1, and after infecting the cells for 6 h at 37°C, the medium was replaced with complete medium for continue culturing for 24 h.
1.3.3. 4 µg/mL puromycin was added to be screened for 5 days, and the surviving cells were the stable cell lines.
1.3.4. The stable cell lines of GSDME were spread in the 96-well plate, the next day, 1 mg/mL doxycycline was added in the experimental group to induce the expression of the target gene, and the 96-well plate was placed in IncuCyte to observe the change of the cellular morphology. After 6 h, the cell supernatant was collected to detect the LDH release.
1.3.5. The results are as shown in FIGs. 1C and 1D, after doxycycline induced the expression of GSDME NT, SW1990 and Capan2 cells underwent pyroptosis, while there was no pyroptosis after the expression of mutant F2A and F2A+K40A NT.

### 2. Functional verification of mutating the sequence recognized by caspase-3 of GSDME protein into the sequence recognized by MMPs

### 2.1. Expression of MMP11 in tumor tissues

The expression of MMP11 in tumor tissues and normal tissues was searched in GEPIA2 database, and the expression of MMP11 in normal human cell lines and human tumor cell lines was detected by the WB method, and the specific steps were as follows:
2.1.1. Cell source and culture: normal human renal epithelial cells HEK-293T, human pancreatic cancer cells Aspc1, Capan2, SW1990, and Panel, and human colorectal cancer cells HCT116 were from the American Type Culture Collection (ATCC) (Cat. No.: CRL-3216; CRL-1682; HTB-80; CRL-2172; CRL-1469; CCL247). Human nasopharyngeal carcinoma cells SUNE2 were constructed by the applicant team laboratory (doi: 10.5732/cjc.01 1. 10317), and human nasopharyngeal carcinoma cells HK1 were a gift from Professor Sai-Wah Tsao of the University of Hong Kong. The above cell lines were all cultured in a DMEM medium (Gbico) containing 10% fetal bovine serum (Invitrogen); and culture conditions were 5% CO₂, 37°C.
2.1.2. The cells were spread in six-well plates in respective, with 500,000 cells per well, and cultured overnight; and the cell lysate was collected for WB experiments, and MMP11 (source: Affinit; Cat. No.: AF0211) and GAPDH served as primary antibodies.
2.1.3. The results are as shown in FIG. 2 and consistent with the results of the GEPIA2 database, normal human renal epithelial cells hardly express MMP11, and human tumor cell lines highly express MMP11.

### 2.2. Construction of GSDME restriction enzyme cutting site mutation vector

The DMPDAAH amino acid sequence recognized by caspase3 in the GSDME protein was mutated to the amino acid sequence recognized by matrix metalloproteinases (MMPs), where the recognition sequence of MMP1 was GPPQAMHA, the recognition sequence of MMP2 was PQGIAGQ, the recognition sequence of MMP9 was SGKIPRRLTA, and the recognition sequence of MMP11 was AANLVR, and these recognition sequences were cloned into the mammalian expression vector pcDNA3.1/pRetro-X 3G (the plasmid was synthesized and constructed by Suzhou Hongxun Biotechnology Co., Ltd.).

### 2.3. Effect of transient transfection of GSDME MMPs plasmid on inducing pyroptosis of tumor cells

HEK293T and SW1990 cells were spread in six-well plates. The next day, pcDNA3.1-GSDME WT/MT/MMP1/MMP2/MMP9/MMP11 plasmids were transfected into the cells using PEI, respectively (the ratio is as above), the change of the cellular morphology was observed with IncuCyte; and after 24 h, the shooting was completed, the cells were collected, and the GSDME NT protein expression was detected by western blot (WB). The results are as shown in FIGs. 3A and 3B, no pyroptosis was observed in HEK293T cells that did not express MMPs, human tumor cells SW1990 that expressed MMPs underwent pyroptosis after being transfected with GSDME MMPs plasmids, and GSDME spliceosome could also be detected in WB results.

### 2.4. Retro-X Tet-On induced expression system to verify the effect of GSDME NT on inducing pyroptosis

HEK-293T and SW1990 cells were used to construct stable cell lines inducibly expressed by GSDME MMP11, the stable cell lines were spread in a 96-well plate, doxycycline was added in the next day to induce expression, IncuCyte was used to observe the change of the cellular morphology, and after 24 h, the shooting was completed and the cell supernatant was used to detect LDH release. The results are as shown in FIGs. 3C and 3D, and pyroptosis occurred after SW1990 induced the expression of GSDME MMP11.

### 3.Construction of TRAP expression vector

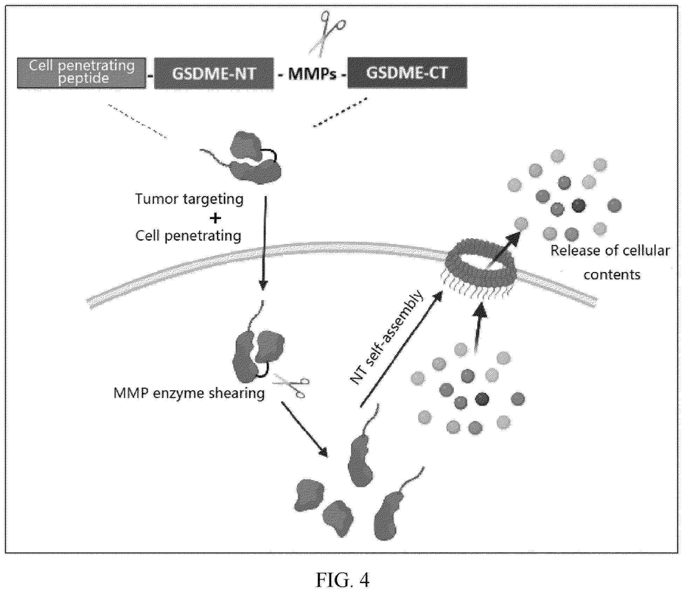

The DNA sequence containing CPP molecular components such as H16, 4*RWYD, nanobody (VHH) or single-chain variable region antibody (ScFv) and GSDNE was synthesized and cloned into the prokaryotic expression vector pET28a (plasmid was synthesized and constructed by Jiangsu Genecefe Biotechnology Co., Ltd.). The synthesized pET28a-CPP-GSDME MMP11 plasmid was transformed into *E.coli* BL (Rosetta) competent cells, and the positive recombinants were screened out through kanamycin-resistant plate and subjected to colony PCR verification and DNA sequencing verification, and the sequencing results were analyzed through NCBI BLAST comparison. Plasmids were stored for long-term at -20°C, and correctly sequenced strains were added to 15% glycerol and stored at -80°C. The vector pET28a has a 6*His tag and can be used for the expression and purification of His protein. The schematic diagram of the construction of TRAP molecules and the schematic diagram of the shearing process are as shown in FIG. 4.

### (II) Expression and purification of TRAP system

1. TRAP was induced and expressed by isopropylthiogalactopyranoside (IPTG), and different IPTG concentrations, action temperatures and action times were used to explore the induction expression conditions. SDS-PAGE and Coomassie Brilliant Blue experiments were subsequently used to determine the optimal expression conditions. In this example, the correctly sequenced bacterial solution was inoculated into the TB medium containing kanamycin resistance (50ng/µL), and shaken and cultured overnight at 37°C. The next day, the bacteria solutions of the activated bacterial strains were accessed to fresh culture mediums in respective at a ratio of 1:100 to expand the culture, the bacteria solutions were shaken and cultured to OD₆₀₀=0.6 at 180 rpm, then cooled to 18°C, isopropyl-β-d-thiogalactoside (IPTG) was added, the final concentration of IPTG was 0.5mM, the induced expression continued for about 18 h under the same conditions, then thalli were centrifuged for 30 min at 5000 rpm and at 4°C, and the thalli were resuspend by 1×PBS buffer and collected again. The collected thalli were resuspended to 10% bacteria solution again by the 1×PBS buffer, frozen and thawed repeatedly in liquid nitrogen at 37°C for 3 times, and then ultrasonicated in an ice bath for 30 min (5s on, 5s off) until the bacterial solution was completely crushed (or a highpressure crusher was directly used, crushed for 3 min at 900 Pa and at 4°C). The thalli were centrifuged for 1 h at 10,000 rpm and at 4°C, the precipitate was discarded, the supernatant was filtered with a 0.22 mm pore size filter membrane, and the supernatant was used for subsequent purification.
2. After resuspended with Ni-NTA agarose gel, the supernatant obtained above was incubated for 2 h at 4°C (incubation with reference to 5g of thalli: 1 mL 50% Ni-NTA agarose gel). The color of Ni-NTA agarose gel changed from blue to brown during the incubation. Centrifugation at 1000 g for 1 min at 4°C, 20 uL of supernatant was taken for subsequent verification of purification effect, most of the supernatant was discarded and only a small amount was kept for resuspending the Ni-NTA agarose gel, then loaded onto the column and washed three times with 1×PBS buffer containing 20 mM imidazole, with twice of column volume each time. 20 uL of the last washing solution was collected, and then 1×PBS buffer containing 300 mM imidazole was used for elution; if the Ni-NTA agarose gel turns blue again, it means that the elution is basically complete. 20 uL of eluate was taken as a sample, and the sample taken from purification was used to detect the purification efficiency of the fusion protein with 10% SDS-PAGE. The eluate was centrifuged at 10,000 g of centrifugal force for 10 min, and the supernatant was subjected to fusion protein concentration by a 10KD ultrafiltration tube (Millipore, Cat. No.: UFC900396), centrifuged at 4500g, ultrafiltrated to 500 µL at 4°C, then the concentrated solution was rinsed three times with 1×PBS, and centrifugation was repeated three times. The concentration of the obtained concentrated protein was measured using a BCA protein concentration detection kit (Thermo Fisher, Cat. No.: 23227) and the concentrated protein was store at -80°C.

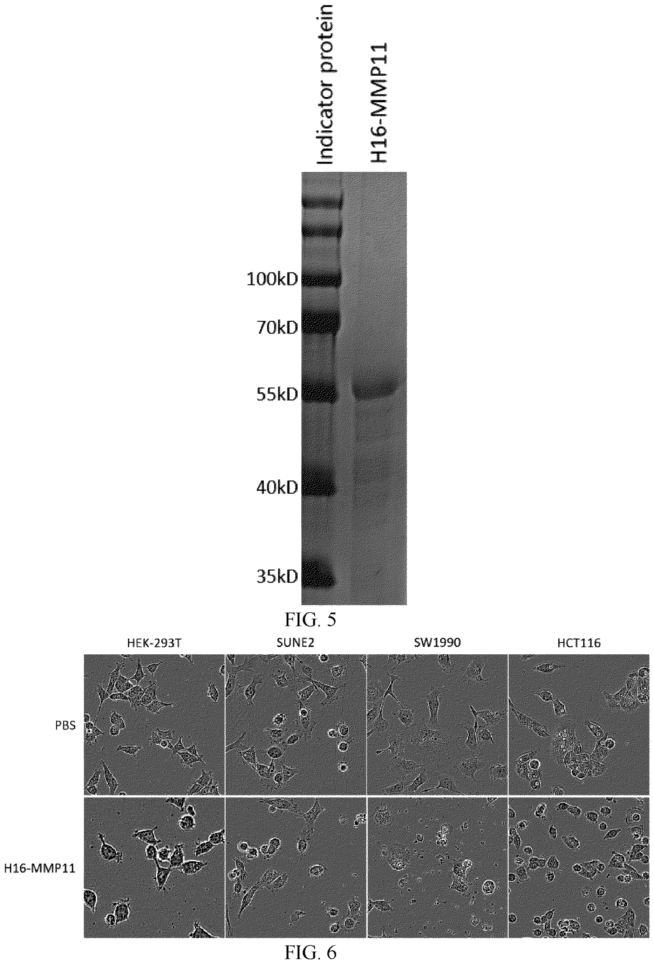

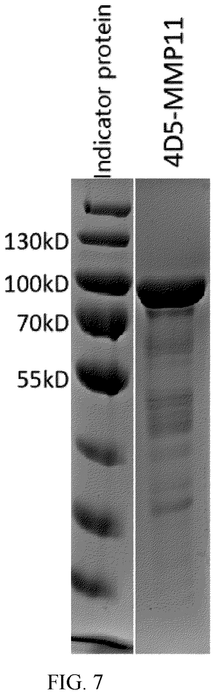

The purification effect of TRAP is as shown in FIGs. 5 and 7, the purity has reached 90% or above and can be used for subsequent experimental verification. The sequence of the GSDME protein is

MFAKATRNFLREVDADGDLIAVSNLNDSDKLQLLSLVTKKKRFWCWQRPKYQFLSLTLGD
VLIEDQFPSPVVVESDFVKYEGKFANHVSGTLETALGKVKLNLGGSSRVESQSSFGTLRKQE
VDLQQLIRDSAERTINLRNPVLQQVLEGRNEVLCVLTQKITTMQKCVISEHMQVEEKCGGI
VGIQTKTVQVSATEDGNVTKDSNVVLEIPAATTIAYGVIELYVKLDGQFEFCLLRGKQGGFE
NKKRIDSVYLDPLVFREFAFIDMPDAAHGISSQDGPLSVLKQATLLLERNFHPFAELPEPQQT
ALSDIFQAVLFDDELLMVLEPVCDDLVSGLSPTVAVLGELKPRQQDLVAFLQLVGCSLQGG
CPGPEDAGSKQLFMTAYFLVSALAEMPDSAAALLGTCCKLQIIPTLCHLLRALSDDGVSDLE
DPTLTPLKDTERFGIVQRLFASADISLERLKSSVKAVILKDSKVFPLLLCITLNGLCALGREHS

(SEQ ID NO: 1).

The amino acid sequence of the GSDME mutant protein (MMP11 for short) after the DMPDAAH amino acid sequence recognized by the caspase3 of the GSDME protein is mutated into the amino acid sequence recognized by the matrix metalloproteinase (MMP11) is:

MFAKATRNFLREVDADGDLIAVSNLNDSDKLQLLSLVTKKKRFWCWQRPKYQFLSLTLGD
VLIEDQFPSPVVVESDFVKYEGKFANHVSGTLETALGKVKLNLGGSSRVESQSSFGTLRKQE
VDLQQLIRDSAERTINLRNPVLQQVLEGRNEVLCVLTQKITTMQKCVISEHMQVEEKCGGI
VGIQTKTVQVSATEDGNVTKDSNVVLEIPAATTIAYGVIELYVKLDGQFEFCLLRGKQGGFE
NKKRIDSVYLDPLVFREFAFIAANLVRGISSQDGPLSVLKQATLLLERNFHPFAELPEPQQTA
LSDIFQAVLFDDELLMVLEPVCDDLVSGLSPTVAVLGELKPRQQQDLVAFLQLVGCSLQGGC
PGPEDAGSKQLFMTAYFLVSALAEMPDSAAALLGTCCKLQIIPTLCHLLRALSDDGVSDLED
PTLTPLKDTERFGIVQRLFASADISLERLKSSVKAVILKDSKVFPLLLCITLNGLCALGREHS
(SEQ ID NO: 2).

The amino acid sequence of H16-MMP11 molecule is: HHHHHHHHHHHHHHMFAKATRNFLREVDADGDLIAVSNLNDSDKLQLLSLVTKKKRFWC WQRPKYQFLSLTLGDVLIEDQFPSPVVVESDFVKYEGKFANHVSGTLETALGKVKLNLGGS SRVESQSSFGTLRKQEVDLQQLIRDSAERTINLRNPVLQQVLEGRNEVLCVLTQKITTMQKC VISEHMQVEEKCGGIVGIQTKTVQVSATEDGNVTKDSNVVLEIPAATTIAYGVIELYVKLDG QFEFCLLRGKQGGFENKKRIDSVYLDPLVFREFAFIAANLVRGISSQDGPLSVLKQATLLLER NFHPFAELPEPQQTALSDIFQAVLFDDELLMVLEPVCDDLVSGLSPTVAVLGELKPRQQQDL VAFLQLVGCSLQGGCPGPEDAGSKQLFMTAYFLVSALAEMPDSAAALLGTCCKLQIIPTLCH LLRALSDDGVSDLEDPTLTPLKDTERFGIVQRLFASADISLERLKSSVKAVILKDSKVFPLLL CITLNGLCALGREHS (SEQ ID NO: 3) (where the single underline is the amino acid sequence of cancer tissue-specific membrane-penetrating peptide H16; and the double underline is the amino acid sequence cleaved by MMP11 recognition enzyme).

The amino acid sequence of 4D5-MMP11 molecule is: DIOMTOSPSSSLSASVGDRVTITCRASODVNTAVAWYOQKPGKAPKLLIYSASFLYSGVPSR FSGSRSGTDFTLTISSLQPEDFATYYCOOHYTTPPTFGOGTKVEIKRTGSTSGKPGSGEGSEV QLVESGGGLVQPGGSLRLSCAASGFNIKDTYIHWVROAPGKGLEWVARIYPTNGYTRYADS VKGRFTISADTSKNTAYLQMNSLRAEDTAVYYCSRWGGDGFYAMDVWGOGTLVTVSSGS MFAKATRNFLREVDADGDLIAVSNLNDSDKLQLLSLVTKKKRFWCWQRPKYQFLSLTLGD VLIEDQFPSPVVVESDFVKYEGKFANHVSGTLETALGKVKLNLGGSSRVESQSSFGTLRKQE VDLQQLIRDSAERTINLRNPVLQQVLEGRNEVLCVLTQKITTMQKCVISEHMQVEEKCGGI VGIQTKTVQVSATEDGNVTKDSNVVLEIPAATTIAYGVIELYVKLDGQFEFCLLRGKQGGFE NKKRIDSVYLDPLVFREFAFIAANLVRGISSQDGPLSVLKQATLLLERNFHPFAELPEPQQTA LSDIFQAVLFDDELLMVLEPVCDDLVSGLSPTVAVLGELKPRQQQDLVAFLQLVGCSLQGGC PGPEDAGSKQLFMTAYFLVSALAEMPDSAAALLGTCCKLQIIPTLCHLLRALSDDGVSDLED PTLTPLKDTERFGIVQRLFASADISLERLKSSVKAVILKDSKVFPLLLCITLNGLCALGREHS (SEQ ID NO: 4) (where the single underline is the amino acid sequence of HER2 single-chain variable region antibody 4D5; and the double underline is the amino acid sequence cleaved by MMP11 recognition enzyme).

### II. Targeting verification of TRAP tumor

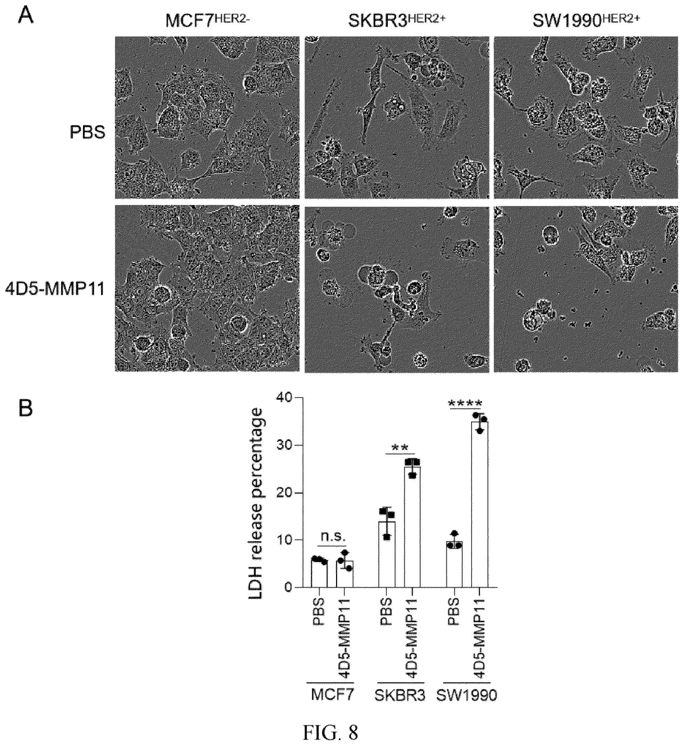

In this example, the targeting of the membrane-penetrating peptide H16 and HER2 single-chain variable region 4D5 carried by TRAP on tumor cells were verified, respectively. The targeting effect of TRAP on tumors was verified using IncuCyte and LDH release experiments and using normal human cells and tumor cells, including tumor cell lines with high and low expressions of HER2.
(1) Normal human renal epithelial cells HEK-293T, human pancreatic cancer cells SW1990 with high expression of HER2, human breast cancer cells MCF7 with low expression of HER2, and human breast cancer cells SKBR3 with high expression of HER2 (from ATCC, Cat. No.: HTB-38, CCL-247) were spread in a 96-well cell culture dish in advance, and 100 µg/mL of H16-MMP11/4D5-MMP11 and their control solvent PBS were added, respectively.
(2) IncuCyte was used to culture and treat the cells for 6 h at 37°C, then 80 µL of the culture supernatant was taken from each well in a new 96-well plate, and a lactate dehydrogenase cytotoxicity detection kit was used to detect the LDH release percentage. The results are as shown in FIGs. 6 and 8, H16-MMP11 can target tumor cells and induce pyroptosis, and 4D5-MMP11 can target tumor cells with high HER2 expression and induce pyroptosis.

### Example 2 Effect verification of GSDME protein cysteine mutation transformation

The design idea of TRAP was explained, the role of TRAP in inducing pyroptosis in tumor cells was verified, and its tumor targeting and pyroptosis-inducing effects were verified in example 1. Because the GSDME protein contains 13 cysteines, it is easy to form disulfide bonds, leading to misfolding of the protein. Therefore, in this example, amino acid mutation is used to reduce the misfolding rate of GSDME protein in vitro, to increase its druggability possibility, and evaluate the effect of cysteine mutation on tumor cell targeting and inducing pyroptosis.

### 1. Effect verification of inducing pyroptosis after GSDME NT cysteine is mutated to alanine or serine

### 1.1. Construction of GSDME NT cysteine mutant plasmid

In the pcDNA3.1-GSDME NT plasmid, all five N-terminal cysteines (Cys, C) were mutated to alanine (Ala, A) or serine (Ser, S), which were named GSDME NT C-A and GSDME NT C-S, respectively.

The amino acid sequence of GSDME NT C-A is: MFAKATRNFLREVDADGDLIAVSN LNDSDKLQLLSLVTKKKRFWAWQRPKYQFLSLTLGDVLIEDQFPSPVVVESDFVKYEGKFA NHVSGTLETALGKVKLNLGGSSRVESQSSFGTLRKQEVDLQQLIRDSAERTINLRNPVLQQV LEGRNEVLAVLTQKITTMQKAVISEHMQVEEKAGGIVGIQTKTVQVSATEDGNVTKDSNVV LEIPAATTIAYGVIELYVKLDGQFEFALLRGKQGGFENKKRIDSVYLDPLVFREFAFI (SEQ I D NO: 5).

The plasmid was synthesized and constructed by Jiangsu Genecefe Biotechnology Co., Ltd. The synthesized plasmid was transformed into DH5α competent cells, and positive recombinants were screened out through an ampicillin-resistant plate and subjected to colony PCR verification and DNA sequencing verification, and the sequencing results were analyzed by NCBI BLAST comparison. Plasmids were stored for long-term at -20°C, and correctly sequenced strains were added to 15% glycerol and stored at -80°C.

### 1.2. Verify the ability to induce pyroptosis after cysteine mutation

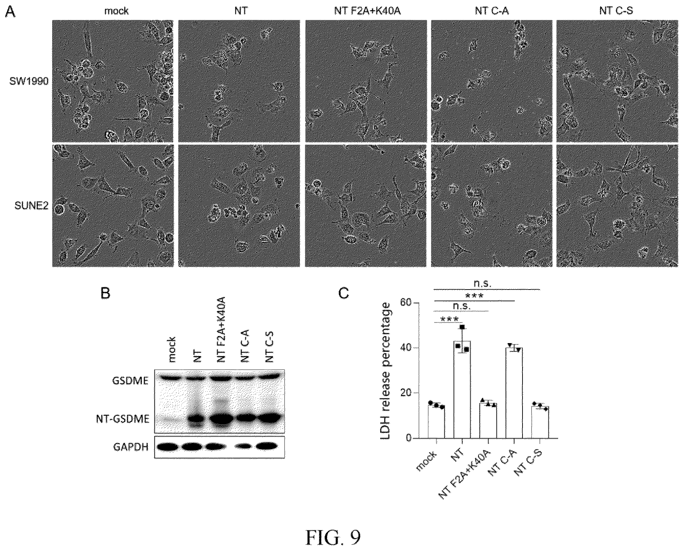

Human pancreatic cancer cells SW1990 and human nasopharyngeal carcinoma cells SUNE2 in the logarithmic growth phase were respectively taken and spread in 96-well plates, each type of cells were divided into 5 groups, with 4 duplicate wells in each group, and cultured overnight. The next day, PEI was used to transfect mock, NT, NT F2A+K40A, C-A or C-S, IncuCyte was used to observe the change of cellular morphology, the shooting was completed after 6 h, the cell supernatant was collected to detect LDH release, and the cell lysate was collected and detected the expression of GSDME NT by WB. The results are as shown in FIG. 9, after mutating the cysteine of GSDME NT to alanine, GSDME NT still has the ability to induce pyroptosis, but after mutating the cysteine to serine, GSDME NT cannot induce pyroptosis. Therefore, we use the protein after mutating cysteine to alanine for subsequent studies.

### 2. Expression and purification of TRAP molecules after cysteine mutation

### 2.1. Construction of TRAP expression vector

The DNA sequence containing H16 and GSDNE MMP11 C-A was synthesized and cloned into the prokaryotic expression vector pET28a (the plasmid was synthesized and constructed by Jiangsu Genecefe Biotechnology Co., Ltd.).

A small molecule ubiquitin-related modification protein (sumo) fusion tag was added before the penetrating peptide H16 component to increase the solubility of the protein and reduce the cytotoxicity of the protein. The synthesized pET28a-sumo-H16-MMP11 C-A plasmid was transformed into *E.coli* BL (Rosetta) competent cells, and the positive recombinants were screened out through the kanamycin-resistant plate, and subjected to colony PCR verification and DNA sequencing verification, and the sequencing results were analyzed through NCBI BLAST comparison. Plasmids were stored for long-term at -20°C, and correctly sequenced strains were added to 15% glycerol and stored at -80°C.

### 2.2. Expression and purification of TRAP after cysteine mutation

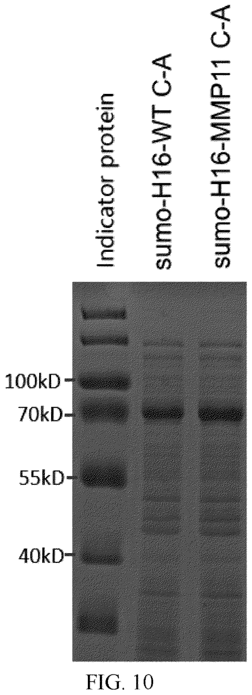

TRAP purification before and after cysteine mutation is consistent. The purification effect is as shown in FIG. 10, and the purity reaches 80% or above and can be used for subsequent experimental verification. The amino acid sequence of GSDNE MMP11 C-A is:

MFAKATRNFLREVDADGDLIAVSNLNDSDKLQLLSLVTKKKRFWAWQRPKYQFLSLTLGD
VLIEDQFPSPVVVESDFVKYEGKFANHVSGTLETALGKVKLNLGGSSRVESQSSFGTLRKQE
VDLQQLIRDSAERTINLRNPVLQQVLEGRNEVLAVLTQKITTMQKAVISEHMQVEEKAGGIV
GIQTKTVQVSATEDGNVTKDSNVVLEIPAATTIAYGVIELYVKLDGQFEFALLRGKQGGFEN
KKRIDSVYLDPLVFREFAFIAANLVRGISSQDGPLSVLKQATLLLERNFHPFAELPEPQQTALS
DIFQAVLFDDELLMVLEPVADDLVSGLSPTVAVLGELKPRQQQDLVAFLQLVGASLQGGAPG
PEDAGSKQLFMTAYFLVSALAEMPDSAAALLGTAAKLQIIPTLAHLLRALSDDGVSDLEDPT
LTPLKDTERFGIVQRLFASADISLERLKSSVKAVILKDSKVFPLLLAITLNGLAALGREHS
(SEQ ID NO: 6).

Where the amino acid sequence of H16-WT C-A MOLECULE is:

HHHHHHHHHHHHHHMFAKATRNFLREVDADGDLIAVSNLNDSDKLQLLSLVTKKKRFWA
WQRPKYQFLSLTLGDVLIEDQFPSPVVVESDFVKYEGKFANHVSGTLETALGKVKLNLGGS
SRVESQSSFGTLRKQEVDLQQLIRDSAERTINLRNPVLQQVLEGRNEVLAVLTQKITTMQKA
VISEHMQVEEKAGGIVGIQTKTVQVSATEDGNVTKDSNVVLEIPAATTIAYGVIELYVKLDG
QFEFALLRGKQGGFENKKRIDSVYLDPLVFREFAFIDMPDAAHGISSQDGPLSVLKQATLLL
ERNFHPFAELPEPQQTALSDIFQAVLFDDELLMVLEPVADDLVSGLSPTVAVLGELKPRQQQ
DLVAFLQLVGASLQGGAPGPEDAGSKQLFMTAYFLVSALAEMPDSAAALLGTAAKLQIIPTL
AHLLRALSDDGVSDLEDPTLTPLKDTERFGIVQRLFASADISLERLKSSVKAVILKDSKVFPL
LLAITLNGLAALGREHS (SEQ ID NO: 7).

The amino acid sequence of H16-MMP11 C-A MOLECULE is:

HHHHHHHHHHHHHMFAKATRNFLREVDADGDLIAVSNLNDSDKLQLLSLVTKKKRFWA
WQRPKYQFLSLTLGDVLIEDQFPSPVVVESDFVKYEGKFANHVSGTLETALGKVKLNLGGS
SRVESQSSFGTLRKQEVDLQQLIRDSAERTINLRNPVLQQVLEGRNEVLAVLTQKITTMQKA
VISEHMQVEEKAGGIVGIQTKTVQVSATEDGNVTKDSNVVLEIPAATTIAYGVIELYVKLDG
QFEFALLRGKQGGFENKKRIDSVYLDPLVFREFAFIAANLVRGISSQDGPLSVLKQATLLLER
NFHPFAELPEPQQTALSDIFQAVLFDDELLMVLEPVADDLVSGLSPTVAVLGELKPRQQQDL
VAFLQLVGASLQGGAPGPEDAGSKQLFMTAYFLVSALAEMPDSAAALLGTAAKLQIIPTLA

HLLRALSDDGVSDLEDPTLTPLKDTERFGIVQRLFASADISLERLKSSVKAVILKDSKVFPLL
LAITLNGLAALGREHS (SEQ ID NO: 8).

The tag sequence is further added and the amino acid sequence of sumo-H16-WT C-A molecule in the control group is set as: 
MSDSEVNQEAKPEVKPEVKPETHINLKVSDGSSEIFFKIKKTTPLRRLMEAFAKRQGKEMDS
LRFLYDGIRIQADQTPEDLDMEDNDIIEAHREQIGGHHHHHHHHHHMFAKATRNFLR
EVDADGDLIAVSNLNDSDKLQLLSLVTKKKRFWAWQRPKYQFLSLTLGDVLIEDQFPSPVV
VESDFVKYEGKFANHVSGTLETALGKVKLNLGGSSRVESQSSFGTLRKQEVDLQQLIRDSA
ERTINLRNPVLQQVLEGRNEVLAVLTQKITTMQKAVISEHMQVEEKAGGIVGIQTKTVQVS
ATEDGNVTKDSNVVLEIPAATTIAYGVIELYVKLDGQFEFALLRGKQGGFENKKRIDSVYLD
PLVFREFAFIDMPDAAHGISSQDGPLSVLKQATLLLERNFHPFAELPEPQQTALSDIFQAVLFD
DELLMVLEPVADDLVSGLSPTVAVLGELKPRQQQDLVAFLQLVGASLQGGAPGPEDAGSKQ
LFMTAYFLVSALAEMPDSAAALLGTAAKLQIIPTLAHLLRALSDDGVSDLEDPTLTPLKDTE
RFGIVQRLFASADISLERLKSSVKAVILKDSKVFPLLLAITLNGLAALGREHS (SEQ ID NO: 9);
 the amino acid sequence of sumo-H16-MMP11 C-A molecule is: 
MSDSEVNQEAKPEVKPEVKPETHINLKVSDGSSEIFFKIKKTTPLRRLMEAFAKRQGKEMDS
LRFLYDGIRIQADQTPEDLDMEDNDIIEAHREQIGGHHHHHHHHHHMFAKATRNFLR
EVDADGDLIAVSNLNDSDKLQLLSLVTKKKRFWAWQRPKYQFLSLTLGDVLIEDQFPSPVV
VESDFVKYEGKFANHVSGTLETALGKVKLNLGGSSRVESQSSFGTLRKQEVDLQQLIRDSA
ERTINLRNPVLQQVLEGRNEVLAVLTQKITTMQKAVISEHMQVEEKAGGIVGIQTKTVQVS
ATEDGNVTKDSNVVLEIPAATTIAYGVIELYVKLDGQFEFALLRGKQGGFENKKRIDSVYLD
PLVFREFAFIAANLVRGISSQDGPLSVLKQATLLLERNFHPFAELPEPQQTALSDIFQAVLFDD
ELLMVLEPVADDLVSGLSPTVAVLGELKPRQQQDLVAFLQLVGASLQGGAPGPEDAGSKQL
FMTAYFLVSALAEMPDSAAALLGTAAKLQIIPTLAHLLRALSDDGVSDLEDPTLTPLKDTER
FGIVQRLFASADISLERLKSSVKAVILKDSKVFPLLLAITLNGLAALGREHS (SEQ ID NO:10)
 (The bullet points below is the sumo tag sequence, and the single underline is the amino acid sequence of the penetrating peptide H16; and the double underline is the amino acid sequence cleaved by MMP11 recognition enzyme).

### 2.3. Effect of TRAP on inducing pyroptosis after cysteine mutation

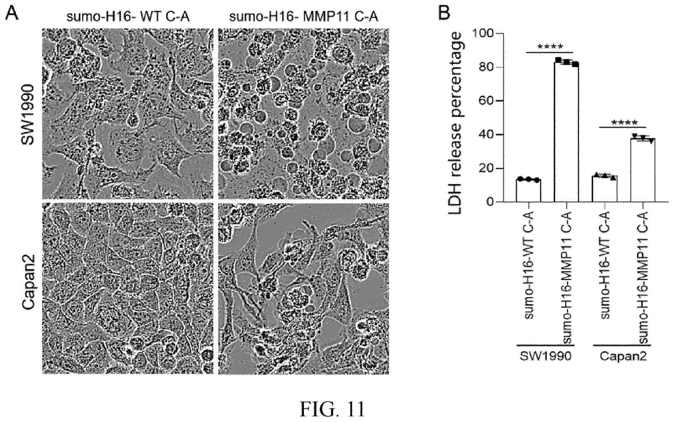

SW1990 and Capan2 in the logarithmic growth phase were taken and spread in six-well plate, respectively, each type of cells was divided into 2 groups and cultured overnight. The next day, 100 µg/mL of sumo-H16-MMP11 C-A and its control group sumo-H16-WT C-A were added in respective, IncuCyte was used to observe the change of cellular morphology, the shooting was completed after 24 h and the cell supernatant was collected to detect LDH release. The results are as shown in FIG. 11, and compared with sumo-H16-WT C-A protein, TRAP protein sumo-H16-MMP11 C-A can significantly induce pyroptosis in tumor cells.

The above data show that after cysteine is mutated to alanine, the effect of TRAP on targeting and inducing pyroptosis in tumor cells is not changed, and the misfolding rate of multiple cysteines in the protein is reduced, thereby improving the druggability of the protein. Therefore, it is proven that TRAP can target tumor tissues and penetrate tumor cells to induce pyroptosis, which will be of great significance for anti-tumor immunotherapy.

The examples of the present invention are described in detail above in combination with the specific embodiments, and however the present invention is not limited the above examples. Under the premise of not departing from the purpose of the present invention, various changes may also be made within the knowledge scope of those skilled in the art. In addition, the examples in the present invention and features in the examples may be combined with each other without conflict.

## Claims

1. A gasdermin-E (GSDME) mutant protein, wherein the GSDME mutant protein based on GSDME is at least one of:
a) a cysteine in SEQ ID NO: 1 is mutated to alanine; and/or
b) a cysteine in SEQ ID NO: 1 is mutated to other amino acids and having the same or similar function as a); and/or
c) a DMPDAAH amino acid sequence recognized by caspase3 in SEQ ID NO: 1 is mutated to an amino acid sequence recognized by matrix metalloproteinases.

2. The GSDME mutant protein according to claim 1, wherein the sequence of the GSDME mutant protein is as shown in SEQ ID NO: 2 or SEQ ID NO: 6.

3. A related biological material of the GSDME mutant protein according to claim 1 or 2, wherein the related biological material is any one of the following (A1) to (A8):
(A1) a nucleic acid molecule encoding the GSDME mutant protein;
(A2) an expression cassette containing the nucleic acid molecule in (A1);
(A3) a recombinant vector containing the nucleic acid molecule in (A1);
(A4) a recombinant vector containing the expression cassette in (A2);
(A5) a recombinant microorganism containing the nucleic acid molecule in (A1);
(A6) a recombinant microorganism containing the expression cassette in (A2);
(A7) a recombinant microorganism containing the recombinant vector in (A3); and
(A8) a recombinant microorganism containing the recombinant vector in (A4).

4. A fusion protein, comprising a GSDME protein and a tumor cell-specific membrane-penetrating peptide, wherein the GSDME protein is the GSDME mutant protein according to any one of claims 1 to 2 or the protein shown in SEQ ID NO: 1.

5. The fusion protein according to claim 4, wherein the tumor cell-specific membrane-penetrating peptide is a protein polypeptide, a single-chain variable region antibody, or a nanobody.

6. The fusion protein according to claim 5, wherein the amino acid sequence of the fusion protein is a) or b):
a) as shown in any one of SEQ ID NO:3-4 or SEQ ID NO:7-8; and
b) the amino acid sequence shown in a) configured to be modified by one or more amino acid substitutions, deletions or additions and has the same or similar function.

7. A related biological material of the fusion protein according to any one of claims 4 to 6, wherein the related biological material is any one of the following (B 1) to (B8):
(B 1) a nucleic acid molecule encoding the fusion protein;
(B2) an expression cassette containing the nucleic acid molecule in (B 1);
(B3) a recombinant vector containing the nucleic acid molecule in (B 1);
(B4) a recombinant vector containing the expression cassette in (B2);
(B5) a recombinant microorganism containing the nucleic acid molecule in (B1);
(B6) a recombinant microorganism containing the expression cassette in (B2);
(B7) a recombinant microorganism containing the recombinant vector in (B3); and
(B8) a recombinant microorganism containing the recombinant vector in (B4).

8. Use of the GSDME mutant protein according to any one of claims 1 to 2 or the related biological material of the GSDME mutant protein according to claim 3 or the fusion protein according to any one of claims 4 to 6 or the related biological material of the fusion protein according to claim 7 in the preparation of a product; and
preferably, the function of the product is any one of (1) to (5):
(1) tumor targeting;
(2) inducing pyroptosis;
(3) tumor cell-specific membrane-penetrating;
(4) activating the body autoimmunity; and
(5) anti-tumor.

9. A product, comprising the GSDME mutant protein according to any one of claims 1 to 2 or the related biological material of the GSDME mutant protein according to claim 3 or the fusion protein according to any one of claims 4 to 6 or the related biological material of the fusion protein according to claim 7; and preferably, the product is a medicine.

10. A combined medicine, comprising the product according to claim 9 and a tumor-targeting drug.
